(19) Europäisches Patentamt
European Patent Office
Office européen des brevets

(11) EP 1 312 652 A1

(12) **DEMANDE DE BREVET EUROPEEN**

(43) Date de publication:
**21.05.2003 Bulletin 2003/21**

(21) Numéro de dépôt: **02292732.1**

(22) Date de dépôt: **31.10.2002**

(51) Int Cl.[7]: **C09D 5/44**, C25D 13/08, C08G 61/12, C12Q 1/00, C09D 7/00

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **14.11.2001 FR 0114702**

(71) Demandeur: **COMMISSARIAT A L'ENERGIE ATOMIQUE**
**75015 Paris (FR)**

(72) Inventeurs:
- **Revol-Cavalier, Frédéric**
**38180 Seyssins (FR)**
- **Blanc, Régis**
**38120 Saint-Egreve (FR)**

(74) Mandataire: **Goulard, Sophie et al**
**Cabinet ORES,**
**36,rue de St Pétersbourg**
**75008 Paris Cedex (FR)**

(54) **Procédé de formation d'une couche de polymères d'épaisseur homogène à la surface d'un support solide, support solide obtenu et ses applications**

(57) La présente invention est relative à un procédé de formation d'une couche de polymères d'épaisseur homogène à la surface d'un support solide, au support solide obtenu en mettant en oeuvre ce procédé, ainsi qu'à ses applications.

EP 1 312 652 A1

## Description

La présente invention est relative à un procédé de formation d'une couche de polymères d'épaisseur homogène à la surface d'un support solide, au support solide obtenu en mettant en oeuvre ce procédé, ainsi qu'à ses applications.

Il existe actuellement un grand intérêt pour les outils d'analyse multiparamétriques connus sous le nom de puces à ADN. Ces outils sont dédiés à l'analyse de macromolécules biologiques telles que par exemple les molécules d'acide nucléique et les protéines.

Ils permettent, par l'intermédiaire d'une couche de polymères liée à au moins une surface d'un support solide, de fixer, par l'intermédiaire de fonctions spécifiques, ces macromolécules biologiques. Les molécules ainsi immobilisées peuvent ensuite être soumises à différentes techniques d'analyse qualitatives et quantitatives ou peuvent également servir de sondes, dans le but de fixer d'autres molécules biologiques d'intérêt, notamment dans le cas de l'hybridation de brins complémentaires d'ADN.

Actuellement, les dépôts de polymères, et en particulier de polymères conducteurs associés à des fonctions spécifiques (biologiques ou chimiques) dans le but de fixer des macromolécules biologiques sont réalisés de différentes manières, incluant les dépôts par voie chimique ou électrochimique.

Les couches de polymères des systèmes actuellement disponibles sur le marché sont plus ou moins épaisses en fonctions des applications recherchées.

Les dépôts de polymères associés à la fixation d'éléments biologiques, sont décrits comme la succession de maillons d'une chaîne formée par un ou plusieurs monomères différents ; certains de ces différents monomères étant eux-mêmes porteurs d'éléments biologiques.

A titre d'illustration, ce genre de construction peut être schématiquement représenté par la structure suivante :

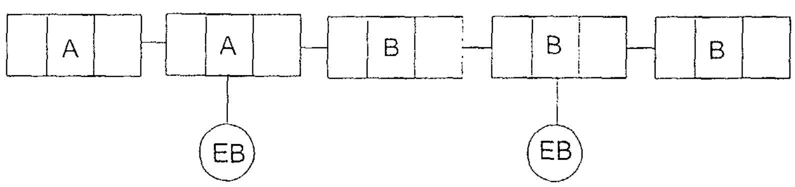


dans laquelle A et B représentent les monomères du polymère et EB représente un élément biologique directement porté par les monomères A et/ou B.

Dans le cas particulier des polymères conducteurs, la polymérisation est réalisée de façon électrochimique. Au cours du processus de polymérisation, la chaîne de polymère conducteur se développe en insérant successivement les différents monomères. Statistiquement, les monomères porteurs d'éléments biologiques se fixent de façon aléatoire à la chaîne, au cours de son élongation. La longueur de la chaîne n'étant pas régulée, l'élongation de celle-ci se poursuit jusqu'à l'arrêt du processus de polymérisation, provoqué, le plus souvent, en fonction du temps, en diminuant le potentiel de dépôt ou bien encore par l'épuisement des monomères. Cette technique ne permet pas de contrôler facilement la longueur des chaînes et conduit, par conséquent, à l'obtention de couches de polymères hétérogènes en terme d'épaisseur.

Par ailleurs, lorsque la polymérisation est réalisée de façon chimique, on mélange par exemple un oxydant chimique aux monomères, au moment de l'emploi, pour initier le processus de polymérisation (polymérisation oxydative). Dans ce cas c'est uniquement le temps pendant lequel on laisse en contact les différents monomères et l'oxydant chimique ou la quantité de monomères initialement présents qui permet de faire varier la longueur finale des chaînes de polymères. Cette technique de polymérisation ne permet donc pas non plus de maîtriser facilement l'épaisseur finale de la couche de polymères formée, ni la quantité d'éléments biologiques éventuellement fixés sur les chaînes ainsi constituées.

Par ailleurs, l'accessibilité des éléments biologiques étant meilleure à la surface des couches de polymères qu'au sein même de cette couche, de nombreux chercheurs se sont employés à diminuer les épaisseurs des couches de polymères, jusqu'à réaliser des dépôts rapides (temps de dépôts court, en général inférieur à 1 seconde).

Ces dépôts rapides nécessitent une grande maîtrise des paramètres mis en jeux au cours de la préparation de la couche de polymères (temps de polymérisation, potentiel de dépôt dans le cas particulier des dépôts électrochimiques, nettoyage des surfaces, etc...). Les dépôts rapides, bien que permettant de conduire à des couches de polymères de faible épaisseur, sont donc délicats à mettre en oeuvre et nécessitent des conditions de réalisation très précises.

D'autre part, lorsque l'on souhaite polymériser plusieurs sites simultanément, les conditions de dépôts font

qu'il est extrêmement difficile de réaliser des épaisseurs de dépôt uniformes sur tous les sites. En effet, les conditions physico-chimiques de la surface du site à polymériser influent sur le démarrage du dépôt, ce qui a pour conséquence, une inhomogénéité dans l'épaisseur des différentes couches de polymères obtenues sur les différents sites à polymériser. Ceci est typiquement le cas lors de l'élaboration des biopuces de type MICAM® telles que décrites par exemple dans la demande de brevet FR-A-2 781 886 ainsi que dans l'article de M.P. Caillat, Sensors and Actuators, B-Chimie, 1999, **61**, 154-162.

C'est donc afin de remédier à l'ensemble de ces inconvénients et de pourvoir à un procédé permettant d'obtenir des supports solides dont au moins une des surfaces est recouverte par une couche polymérique homogène et de faible épaisseur, que les Inventeurs ont mis au point ce qui fait l'objet de la présente invention.

Les Inventeurs ont notamment mis en évidence, de façon surprenante et inattendue, qu'il était possible de préparer de tels supports en utilisant un mélange de différents monomères, ledit mélange étant constitué à au moins 50 % par des monomères comportant une fonction chimique apte à bloquer la réaction de polymérisation.

La présente invention a donc pour premier objet un procédé de formation d'une couche polymérique d'épaisseur homogène sur au moins une partie de l'une des surfaces d'un support solide, caractérisé par le fait que ladite couche polymérique est obtenue par copolymérisation d'une solution de monomères renfermant des monomères porteurs d'une fonction apte à bloquer la réaction de copolymérisation, et dans laquelle le nombre de monomères porteurs de ladite fonction représente au moins 50 % du nombre total de monomères présents au sein de ladite solution.

Les Inventeurs ont en effet démontré que le procédé conforme l'Invention permet d'autoréguler le dépôt des monomères et par-là même, l'élongation des chaînes de polymères, en bloquant successivement l'élongation des chaînes en cours de polymérisation. Ceci permet de diminuer fortement la vitesse de l'élongation des chaînes et d'obtenir des conditions de dépôts plus souples sur les sites que l'on souhaite recouvrir. De façon surprenante, ce procédé permet désormais d'obtenir des couches de polymères de faible épaisseur et remarquablement homogènes.

De plus, lorsque l'on désire réaliser la polymérisation de plusieurs sites simultanément, par exemple dans le cas des biopuces possédant plusieurs milliers de sites différents, le démarrage de la polymérisation peut être différent entre deux sites du fait des conditions physico-chimiques des surfaces. Dans ce cas, le procédé conforme à l'invention permet de freiner la croissance de la couche par blocage successif des chaînes au cours de leur élongation et de réduire l'écart dû au démarrage différé entre deux sites au fur et à mesure de la croissance du dépôt initial. Au final, l'écart d'épaisseur entre les différents sites est réduit, la biopuce présente des épaisseurs de polymères uniformes sur chacun de ses différents sites.

Le procédé conforme à l'Invention permet également de limiter et de maîtriser la quantité d'éléments biologiques fixés sur une chaîne de polymères.

Selon une forme de réalisation avantageuse de l'invention le nombre de monomères porteurs de ladite fonction représente de préférence au moins 60 %, et encore plus préférentiellement 60 à 95 % du nombre total de monomères présents dans ladite solution.

Le procédé conforme à l'Invention permet de contrôler la croissance de la couche de polymères dont l'épaisseur est alors plus homogène. Plus précisément, le procédé conforme à l'Invention permet de diminuer la vitesse d'obtention de la couche de polymère jusqu'à un facteur 10 environ. L'écart type (E) de la mesure d'épaisseur (effectuée sur des couches de polymères dont l'épaisseur peut varier entre quelques nanomètres et quelques dizaine de nanomètres) peut être calculé selon l'équation suivante :

$$E = \sqrt{\frac{1}{n}\sum (X_i - X_m)^2}$$

dans laquelle n représente le nombre de mesure, $X_1$ représente la valeur en Angströms de l'épaisseur au point de mesure et $X_m$ représente la valeur moyenne de l'épaisseur.

Cet écart type E passe de 90 pour une couche polymérique obtenue en mettant uniquement en oeuvre des monomères non fonctionnalisés à 9 seulement si l'on utilise un mélange de monomères renfermant 85 % environ de monomères fonctionnalisés par une fonction apte à bloquer la réaction de copolymérisation par rapport au nombre total de monomères.

Selon l'Invention, la nature même des monomères n'est pas critique.

Les monomères pouvant être utilisés selon le procédé conforme à l'invention sont en fait choisis en fonction du type d'application envisagée et notamment en fonction du type de macromolécules que l'on souhaite fixer.

Ces monomères peuvent notamment être choisis parmi les pyrroles, les anilines, les thiophènes et les azulènes.

La réaction de copolymérisation peut être déclenchée, en fonction de la nature des monomères, soit par un oxydant chimique, soit par un signal électrique (électropolymérisation) dans le cas des monomères conducteurs.

Parmi les oxydant chimiques, on peut notamment citer le chlorure ferrique, le chlorure cuivrique, le chlorure de molybdène (V) et le trichlorure de ruthénium.

Selon une forme de réalisation préférée de l'Invention, lesdits monomères sont choisis parmi les monomères pyrrole et la réaction de copolymérisation est une réaction d'électropolymérisation.

Parmi les fonctions aptes à bloquer la réaction de copolymérisation, on peut notamment citer les fonctions aldéhyde, nitrile, ester, alkyle et aryle.

De manière avantageuse, ladite fonction est de préférence choisie parmi les fonctions permettant d'assurer l'accrochage d'un élément biologique ou biochimique contenant par exemple une fonction amine, tel que par exemple un brin d'ADN, une biotine, une streptavidine ou un anticorps.

A titre d'exemple. et lorsque le monomère est un pyrrole porteur d'une Fonction aldéhyde, l'accrochage de l'élément biologique pourra se faire directement sur la fonction apte à bloquer la réaction de polymérisation, et ce selon la réaction suivante:

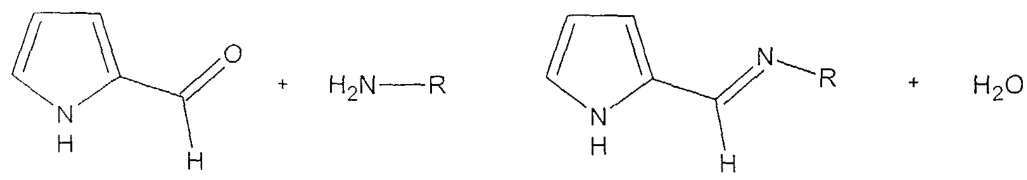

dans laquelle R représente un élément biologique ou biochimique.

On obtiendra ainsi un support solide comportant une couche polymérique dans laquelle l'élément biologique ou biochimique sera fixé en bout de chaîne selon la formule suivante :

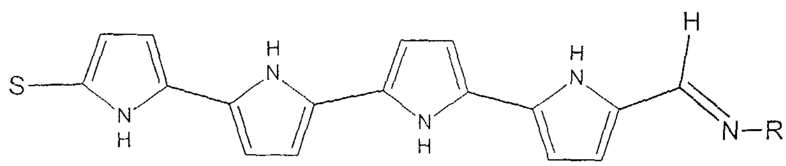

dans laquelle R représente un élément biologique ou biochimique et S correspond à la surface d'un support solide.

Ce procédé permet donc également d'obtenir des supports solides dans lesquels la couche de polymères est particulièrement homogène et qui présentent l'avantage supplémentaire de comporter des éléments biologiques ou biochimiques très accessibles. Cette accessibilité est notamment intéressante dans le cas où l'on souhaite réaliser des réactions d'hybridation de brins d'ADN.

Les supports solides utilisables conformément à l'invention sont de préférence choisis parmi les supports métalliques, tels que les supports en or, les supports en verre ou en matériau plastique.

L'Invention a donc également pour objet le support solide obtenu en mettant en oeuvre le procédé décrit cidessus. Ce support solide comporte, sur au moins une partie de l'une de ses surfaces, une couche de polymère dont l'épaisseur est de préférence comprise entre environ quelques nanomètres et quelques dizaines de nanomètres.

L'invention a également pour objet l'utilisation d'au moins un support solide conforme à l'invention pour l'isolement, la séparation, et/ou l'analyse d'éléments biologiques et biochimiques. Ainsi, l'Invention trouve une application dans la réalisation de puces biologiques de type MICAM® améliorées mais aussi de puces biologiques dont le support solide est en verre ou en matériau plastique.

Outre les dispositions qui précèdent, l'Invention comprend encore d'autres dispositions qui ressortiront de la description qui va suivre, qui se réfère à un exemple de mesure de la vitesse de formation d'une couche de polypyrrole à la surface d'un support solide en fonction du rapport pyrrole/pyrrole porteur d'une fonction aldéhyde, à un exemple de mesure de l'épaisseur et de l'homogénéité d'une couche polymérique à la surface d'un support solide en fonction du rapport pyrrole/pyrrole porteur d'une fonction aldéhyde ainsi qu'aux figures annexées dans lesquelles :

- la figure 1 représente la vitesse de formation de la couche de polymère (en pourcentage de la vitesse initiale) en fonction du pourcentage de monomère pyrrole-2-carboxaldéhyde ;
- la figure 2 représente l'écart type E de la mesure d'épaisseur en fonction du pourcentage décroissant de pyrrole-2-carboxaldéhyde présent dans chaque solution de monomères.

**EXEMPLE 1 : Mesure de la vitesse de formation d'une couche de polymère de type polypyrrole en fonction du pourcentage de monomères pyrrole-2-carboxaldéhyde présents au sein d'un mélange de monomères pyrrole/pyrrole-2-carboxaldéhyde.**

1) Matériel et méthode

On prépare 9 solutions de monomères pyrrole/pyrrole-2-carboxaldéhyde (Aldrich) comprenant de 10 à 90 % de monomères pyrrole-2-carboxaldéhyde par rapport au nombre total de monomères pyrrole/pyrrole-2-carboxaldéhyde (à une concentration de 1 molaire dans l'acétonitrile).

Deux solutions de monomères (à une concentration de 1 molaire dans l'acétonitrile) constituées respectivement uniquement de monomères pyrrole ou de monomères pyrrole-2-carboxaldéhyde ont été également préparées.

Les dépôts des différentes solutions de monomères ainsi préparées sont effectués sur des plots en or ayant tous la même dimension. Le potentiel de dépôt est fixé à 1,1 V (électrode au calomel saturée). Pour chaque solution, le dépôt est réalisé jusqu'à obtenir une charge de 5 mC. Le temps nécessaire pour atteindre cette charge est relevé. Le courant moyen du dépôt est calculé en divisant la charge totale par le temps du dépôt. La réaction de polymérisation est initiée par voie électrochimique. L'électrolyte support utilisé est du $LiClO_4$ aqueux à 0,1 M. La vitesse de formation de la couche polymérique est donnée par le courant moyen du dépôt.

2) Résultats

En comparant entre eux les différents courants moyens obtenus, et en fixant à 100 % la vitesse maximale obtenue, on obtient une courbe représentant la vitesse de formation de la couche de polymère en fonction du pourcentage de monomères pyrrole-2-carboxaldéhyde présent dans chaque solution de monomères (figure 1).

Ces résultats montrent qu'une solution de monomères pyrrole renfermant au moins 50 % en nombre de monomères pyrrole-2-carboxaldéhyde, conduit à une diminution d'environ 10 % de la vitesse de formation de la couche de polymère. Lorsque le pourcentage de monomères pyrrole-2-carboxaldéhyde est compris entre 60 et 70 %, alors la vitesse de formation de la couche de polymère est réduite d'environ 50 %.

**EXEMPLE 2 : Mesure de l'homogénéité d'une couche de polymère de type polypyrrole en fonction du pourcentage de monomères pyrrole-2-carboxaldéhyde présent au sein d'un mélange de monomères pyrrole/pyrrole-2-carboxaldéhyde.**

1) Matériel et méthode

On prépare 4 solutions de monomères pyrrole/pyrrole-2-carboxaldéhyde (Aldrich) comprenant de 46 à 86 % (46 % ; 66 %; 78 % et 86 %) de monomères pyrrole-2-carboxaldéhyde par rapport au nombre total de monomères pyrrole/pyrrole-2-carboxaldéhyde (à une concentration de 1 molaire dans l'acétonitrile).

Deux solutions de monomères (à une concentration de 1 molaire dans l'acétonitrile) constituées respectivement uniquement de monomères pyrrole ou de monomères pyrrole-2-carboxaldéhyde ont été également préparées.

Les dépôts des différentes solutions de monomères ainsi préparées sont effectués selon la méthode décrite ci-dessus à l'exemple 1.

Pour chaque solution, le dépôt est réalisé jusqu'à obtenir une charge de 15 mC.

L'épaisseur et l'homogénéité du dépôt sont ensuite mesurées à l'aide d'un interféromètre optique de marque NANOSPEC®. L'indice de réfraction de la couche est fixé arbitrairement à 1,7.

Chaque dépôt est mesuré en 5 points.

L'homogénéité du dépôt est caractérisée par l'écart type (E) des mesures d'épaisseurs obtenues.

Les résultats obtenus sont reportés sur la figure 2 qui représente l'écart type E de la mesure d'épaisseur en fonction du pourcentage décroissant de pyrrole-2-carboxaldéhyde présent dans chaque solution de monomères. Ces résultats montrent que l'écart type E est diminué d'un facteur de 2 environ lorsque le mélange de monomères utilisé renferme, conformément à l'invention, 66 % de monomères porteurs d'une fonction apte à bloquer la réaction de copolymérisation. Ces résultats montrent également que l'écart type E passe d'environ 95 à seulement 9 environ lorsque le pourcentage de monomères pyrrole-2-carboxaldéhyde passe de 0 % à 85 %.

Ces résultats sont significatifs de la très bonne homogénéité de la couche polymérique obtenue en mettant en oeuvre le procédé conforme à la présente invention.

**Revendications**

1. Procédé de formation d'une couche polymérique d'épaisseur homogène sur au moins une partie de l'une des surfaces d'un support solide, **caractérisé par le fait que** ladite couche polymérique est obtenue par copolymérisation d'une solution de monomères renfermant des monomères porteurs d'une fonction apte à bloquer la réaction de copolymérisation, et dans laquelle le nombre de monomères porteurs de ladite fonction représente au moins 50 % du nombre total de monomères présents au sein de ladite solution.

2. Procédé selon la revendication 1, **caractérisé par le fait que** le nombre de monomères porteurs de ladite fonction représente au moins 60 % du nombre total de monomères présents dans ladite solution.

3. Procédé selon la revendication 2, **caractérisé par le fait que** le nombre de monomères porteurs de ladite fonction représente de 60 à 95 % du nombre total de monomères présents dans ladite solution.

4. Procédé selon l'une quelconque des revendications précédentes, **caractérisé par le fait que** les monomères sont choisis parmi les pyrroles, les anilines, les thiophènes et les azulènes.

5. Procédé selon la revendication 4, **caractérisé par le fait que** les monomères sont choisis parmi les pyrroles et que la réaction de copolymérisation est une réaction d'électropolynérisation.

6. Procédé selon l'une quelconque des revendications précédentes, **caractérisé par le fait que** les fonctions aptes à bloquer la réaction de copolymérisation sont choisies parmi les fonctions aldéhyde, nitrile, ester, alkyle et aryle.

7. Support solide **caractérisé par le fait qu'**il est obtenu selon le procédé tel que défini à l'une quelconque des revendications 1 à 6.

8. Utilisation d'au moins un support solide tel que défini à la revendication 7, pour l'isolement, la séparation, et/ou l'analyse d'éléments biologiques et biochimiques.

9. Utilisation d'au moins un support solide tel que défini à la revendication 7, pour la réalisation de puces biologiques.

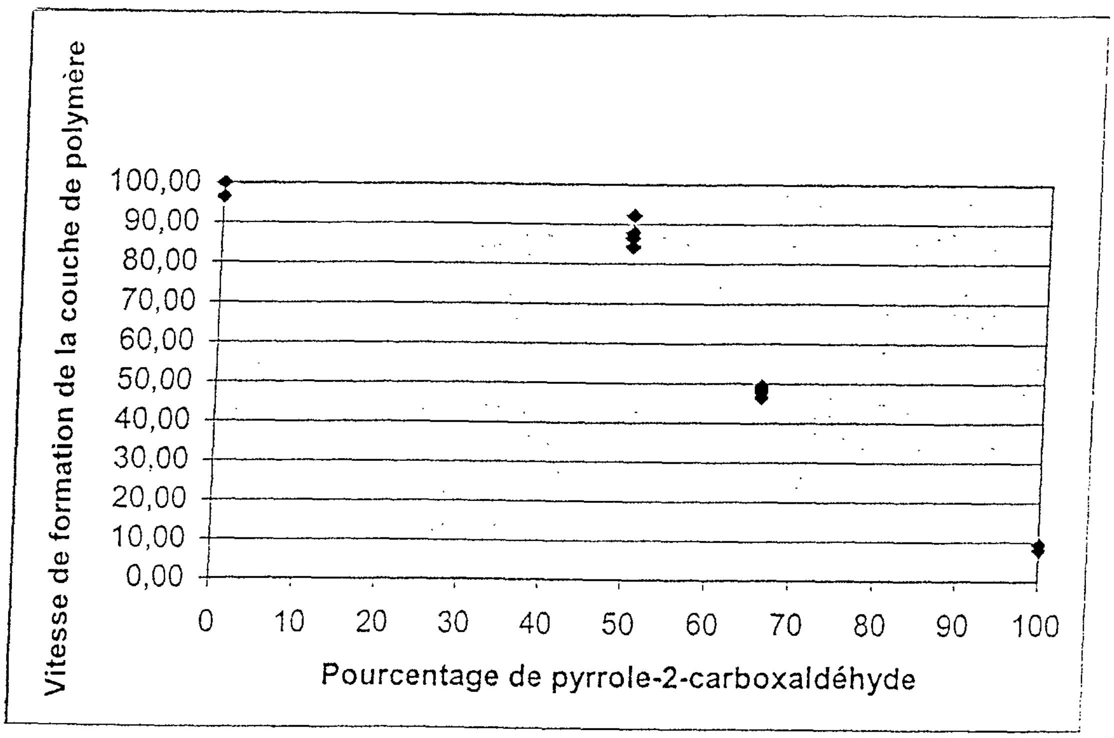
Vitesse de formation de la couche de polymère
100,00
90,00
80,00
70,00
60,00
50,00
40,00
30,00
20,00
10,00
0,00
0
10
20
30
40
50
60
70
80
90
100
Pourcentage de pyrrole-2-carboxaldéhyde

Fig. 1

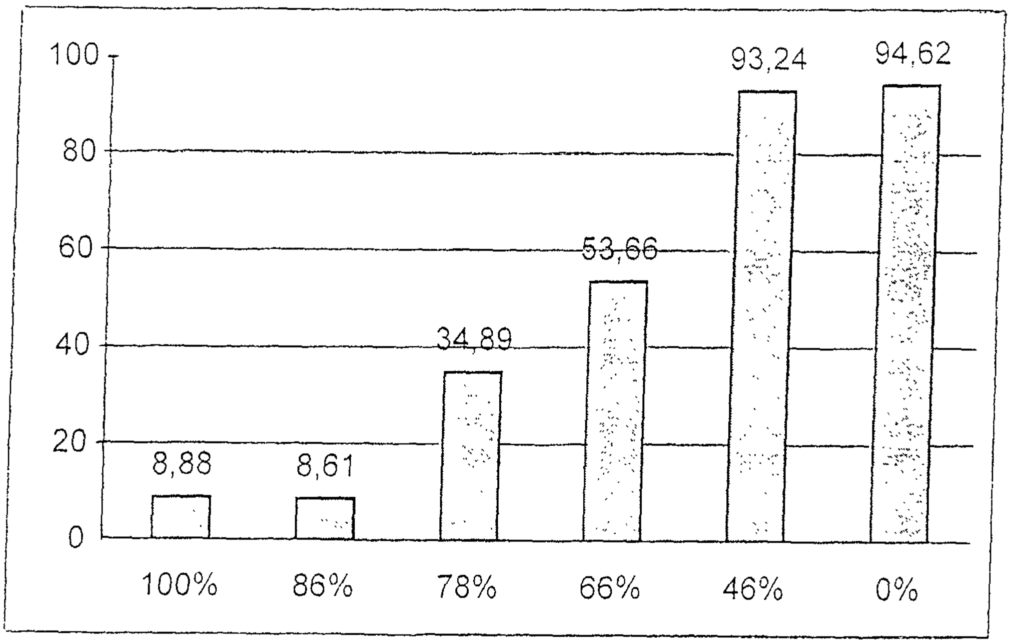


Fig. 2

Office européen des brevets

# RAPPORT DE RECHERCHE EUROPEENNE

Numéro de la demande
EP 02 29 2732

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 5 837 859 A (ROGET ANDRE ET AL) 17 novembre 1998 (1998-11-17)<br>* colonne 5, ligne 33,34 *<br>* colonne 5, ligne 45-54; revendications * | 1,4,5, 7-9 | C09D5/44<br>C25D13/08<br>C08G61/12<br>C12Q1/00<br>C09D7/00 |
| A | US 5 522 981 A (LACAZE PIERRE C ET AL) 4 juin 1996 (1996-06-04)<br>* abrégé; exemple 7 * | 1,4,5,7 | |
| A | FR 2 679 240 A (PECHINEY UGINE KUHLMANN ;CITROEN SA (FR); CENTRE NAT RECH SCIENT () 22 janvier 1993 (1993-01-22)<br>* abrégé * | 1 | |
| A | EP 0 038 244 A (COMMISSARIAT ENERGIE ATOMIQUE) 21 octobre 1981 (1981-10-21)<br>* abrégé * | 1 | |
| A | WO 91 06625 A (PITTNER FRITZ ;SCHALKHAMMER THOMAS (AT); MANN BUXBAUM EVA (AT); MA) 16 mai 1991 (1991-05-16)<br>* abrégé * | 8,9 | |

DOMAINES TECHNIQUES RECHERCHES (Int.Cl.7)

C09D
C08G
C12Q
C25D
C03C
C08J

Le présent rapport a été établi pour toutes les revendications

| Lieu de la recherche | Date d'achèvement de la recherche | Examinateur |
|---|---|---|
| LA HAYE | 18 février 2003 | Girard, Y |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 02 29 2732

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-02-2003

| Document brevet cité au rapport de recherche | | Date de publication | Membre(s) de la famille de brevet(s) | | Date de publication |
|---|---|---|---|---|---|
| US 5837859 | A | 17-11-1998 | FR | 2703359 A1 | 07-10-1994 |
| | | | AT | 159028 T | 15-10-1997 |
| | | | DE | 69406119 D1 | 13-11-1997 |
| | | | DE | 69406119 T2 | 26-03-1998 |
| | | | DK | 691978 T3 | 25-05-1998 |
| | | | EP | 0691978 A1 | 17-01-1996 |
| | | | ES | 2110228 T3 | 01-02-1998 |
| | | | WO | 9422889 A1 | 13-10-1994 |
| | | | GR | 3025738 T3 | 31-03-1998 |
| | | | JP | 3247957 B2 | 21-01-2002 |
| | | | JP | 8508311 T | 03-09-1996 |
| | | | US | 6197949 B1 | 06-03-2001 |
| US 5522981 | A | 04-06-1996 | FR | 2714077 A1 | 23-06-1995 |
| | | | AT | 177444 T | 15-03-1999 |
| | | | DE | 69416971 D1 | 15-04-1999 |
| | | | DE | 69416971 T2 | 07-10-1999 |
| | | | EP | 0659794 A1 | 28-06-1995 |
| | | | ES | 2131654 T3 | 01-08-1999 |
| | | | JP | 3186484 B2 | 11-07-2001 |
| | | | JP | 7331491 A | 19-12-1995 |
| FR 2679240 | A | 22-01-1993 | FR | 2679240 A1 | 22-01-1993 |
| EP 0038244 | A | 21-10-1981 | FR | 2480314 A1 | 16-10-1981 |
| | | | DE | 3171663 D1 | 12-09-1985 |
| | | | EP | 0038244 A1 | 21-10-1981 |
| WO 9106625 | A | 16-05-1991 | AT | 397512 B | 25-04-1994 |
| | | | AT | 249289 A | 15-09-1993 |
| | | | WO | 9106625 A1 | 16-05-1991 |
| | | | AU | 6506390 A | 31-05-1991 |
| | | | EP | 0493511 A1 | 08-07-1992 |
| | | | JP | 5501198 T | 11-03-1993 |

EPO FORM P0460

Pour tout renseignement concernant cette annexe : voir Journal Officiel de l'Office européen des brevets, No.12/82